# NEW EUROPEAN PATENT SPECIFICATION

(11) **EP 0 533 227 B2**
(45) Date of publication and mention of the opposition decision: **03.11.1999**
(45) Mention of the grant of the patent: 17.04.1996
(21) Application number: 92202516.8
(22) Date of filing: 17.08.1992
(51) Int. Cl.: C07C 1/04, C10G 2/00, B01J 37/18

(54) **Process for the activation of a Fischer-Tropsch catalyst and the activated catalyst**
Verfahren zur Aktivierung eines Fischer-Tropsch Katalysators und der aktivierte Katalysator
Procédé pour l'activation d'un Fischer-Tropsch catalyseur et le catalyseur activé

(30) Priority: 20.08.1991 GB 9117899
(43) Date of publication of application: 24.03.1993
(73) Proprietor: SHELL INTERNATIONALE RESEARCH MAATSCHAPPIJ B.V., 2596 HR Den Haag (NL)
(72) Inventor: Eilers, Jacobus, NL-1031 CM Amsterdam (NL); van Erp, Willibrord Adelbert, NL-1031 CM Amsterdam (NL); Tijm, Petrus Jacobus Adrianus, NL-1031 CM Amsterdam (NL); Ansorge, Joachim, NL-2596 HR The Hague (NL)

(56) References cited:
- EP-A- 221 598
- EP-A- 0 152 652
- EP-A- 0 168 894
- WO-A-92/06784
- US-A- 2 289 731
- US-A- 2 644 829
- US-A- 4 413 064
- US-A- 4 492 774
- US-A- 4 670 414
- US-A- 4 729 891
- Ind. Eng. Chem., December 1928, pp. 1341-1348
- Catalysis, vol IV (1956), pp. 82-86
- Storch et al "The Fischer-Tropsch and Related Syntheses", John Wiley & Sons (1951), p. 147
- Roe et al "Role of supports for cobalt-based catalysis used in Fischer-Tropsch synthesis of hydrocarbons" (1988), Elsevier, p. 509

## Description

The present invention relates to a process for the activation of a catalyst, in particular to a process for the activation of a catalyst of use in Fischer-Tropsch synthesis.

The preparation of hydrocarbons from a mixture of hydrogen and carbon monoxide at elevated temperature and pressure in the presence of a catalyst is referred to in the literature as the Fischer-Tropsch hydrocarbon synthesis.

Catalysts used in Fischer-Tropsch synthesis typically comprise one or more metals from Group VIII of the Periodic Table, optionally together with one or more promoters, and a support material or carrier. Particular interest exists in Fischer-Tropsch catalysts which comprise cobalt, especially in catalysts comprising cobalt in association with one or more promoters. Cobalt-containing catalysts have found particular application in the Fischer-Tropsch synthesis of hydrocarbons, yielding products consisting virtually completely of unbranched hydrocarbons with a high degree of selectivity to C₅+ hydrocarbons.

Before a catalyst can be used in Fischer-Tropsch synthesis, it must first be activated. Activation is effected by contacting the catalyst with a hydrogen-containing gas. The action of the activation step is to reduce the oxides of the catalytically active metal and oxides of other metals present as promoters in the catalyst. Such activation procedures, applicable to the activation of fresh catalyst and also in the procedures for regenerating or reactivating exhausted catalyst, are known in the art.

Thus, US Patent No. 2,289,731 (US 2,289,731) discloses a process for the reactivation (regeneration) of exhausted Fischer-Tropsch catalysts in which the catalyst is contacted with hydrogen to remove paraffinic hydrocarbons and other deposits from the catalyst particles. US 2,289,731 further discloses that it is of advantage to expose the catalyst particles to the oxidizing action of an oxygen-containing gas prior to being treated with hydrogen.

European Patent Application publication No. 0 168.894 (EP-A-0 168 894) discloses a process for the activation of a cobalt/zirconium/silica catalyst in which the catalyst particles are contacted with a hydrogen-containing gas at a temperature of between 200 and 350 °C and a hydrogen partial pressure between 0.001 and 75 bar, the hydrogen partial pressure being increased gradually or stepwise from an initial value to a final value such that the final value is at least five times the initial value.

US Patent No. 4,670,414 (US 4,670,414) discloses a process for the conversion of synthesis gas into hydrocarbons with a catalyst prepared by subjecting a cobalt carbonyl-impregnated alumina or silica support to an activation procedure comprising the steps, in sequence, of (A) reduction in hydrogen, (B) oxidation in an oxygen-containing gas, and (C) reduction in hydrogen, the activation procedure being conducted at a temperature below 500 °C. The catalyst is preferably slowly reduced in the presence of hydrogen. The reduction step can be conducted initially using a gaseous mixture comprising 5% hydrogen and 95% nitrogen, and thereafter, the concentration of hydrogen can be gradually increased until pure hydrogen is obtained. The reduced catalyst is then passivated at ambient temperature by contact with diluted air, after which the catalyst is slowly heated in diluted air to a temperature of from about 300 °C to about 350 °C. The thus oxidized catalyst is then reduced in the aforementioned manner. A similar activation procedure is disclosed in US Patent No. 4,413,064.

In US 4,670,414 it is stated that the flow of reducing gas during the reduction stages of the procedure must be high enough so that any water formed has a partial pressure in the offgas of below 1%, in order to avoid excessive steaming of the exit end of the catalyst bed. Thus, to keep the water partial pressure to the required low level requires either the provision of equipment capable of handling a high throughput of gas, or operation of the reduction stages at low pressures, in turn requiring longer reduction periods.

Finally, US Patent No. 2,644,829 (US 2,644,829) discloses a process for the start-up or conditioning of a catalyst in a Fischer-Tropsch synthesis process in which, at the start of the synthesis the catalyst is contacted with a mixture of hydrogen and carbon monoxide at a pressure and a gas space velocity substantially lower than the pressure and gas space velocity normally used to effect the synthesis reaction. The pressure and space velocity are then gradually increased until normal process operating conditions are achieved. The effect of this conditioning procedure is described in US 2,644,829 as being to reduce the initial undesirably high level of activity of the catalyst. Thereafter, the synthesis process is continued under normal operating conditions with the catalyst in its state of reduced activity.

It has been found that, during the activation of a Fischer-Tropsch catalyst, the hydrogen-containing gas must be contacted with the catalyst at very high gas space velocities in order to achieve optimum activation. However, the design and construction of commercial scale reactors often places very severe constraints on the maximum permissable pressure drop across a catalyst bed. These constraints in turn mean that the very high gas space velocities desirable during activation cannot be applied without expensive compressor apparatus, if at all. Accordingly, relatively low gas space velocities must be used during activation. This, however, in turn often results in the activation procedures taking many hours to effectively reduce, and hence activate, the catalyst. Therefore, there is a need for an activation procedure which is capable of effectively activating the catalyst rapidly at low gas space velocities.

Most surprisingly, it has now been found that the operation of an activation procedure for a Fischer-Tropsch catalyst in which the catalyst is reduced by contact with a mixture of hydrogen and one or more inert gases under a regime of increasing hydrogen concentration and increasing gas space velocity yields a catalyst having most advantageous properties. In particular, catalysts activated by the aforementioned procedure can exhibit a significantly increased activity and a markedly higher selectivity to C₅+ hydrocarbons than equivalent catalysts activated by means of prior art processes. Further, the aforementioned advantages may be obtained very rapidly by operation at relatively low gas space velocities to meet the pressure drop requirements of commercial plants.

Accordingly, the present invention provides a process for the activation of a Fischer-Tropsch catalyst comprising reducing the catalyst by contact with a mixture consisting of hydrogen and one or more inert gases, the hydrogen concentration and the space velocity of the gas expressed as Nl/l/h contacting the catalyst increasing step-wise or continuously during the activation, wherein the hydrogen content of the gas mixture is increased from 0.5% v/v or less to 100% v/v.

An example of a suitable inert gas is nitrogen.

As discussed in detail hereinafter, the hydrogen concentration in the mixture may range from 0.5% v/v or less to 100% v/v.

The action of the gas mixture on the catalyst during the activation procedure is to reduce oxides of the catalytically active metal, and other metals present. In this respect, the activation of the catalyst by contact with a mixture of hydrogen and one or more inert gases to effect the reduction is to be distinguished from the process of contacting the catalyst with a gas containing hydrogen and other reactants, such as synthesis gas, to prepare hydrocarbons. The reduction of the metal oxides in the catalyst yields water. As described in US 4,670,414, it is important that the partial pressure of water in the gas contacting the catalyst is kept at a low level, in order to avoid damaging the catalyst. Thus, the water partial pressure in the gas leaving the catalyst bed is preferably maintained at a level below 200 mbar, more preferably below 100 mbar. The maximum water partial pressure possible without damaging the catalyst will vary according to the specific catalyst selected, it having been found that some catalysts are more tolerant to the presence of water than others. When operating with a catalyst having a particularly low tolerance to the presence of water, it may be necessary to maintain the water partial pressure at 50 mbar, or even considerably lower. The tolerance of the catalyst to the presence of water can readily be determined for a given catalyst by determining the activity of the catalyst in Fischer-Tropsch synthesis after contact with varying amounts of water in the activation procedure.

In order to keep the water partial pressure to a minimum, it is preferable to contact the catalyst initially with a mixture containing a high level of inert gas and, thereafter, increase the hydrogen content of the mixture stepwise or continuously whilst monitoring the water content of the gas leaving the catalyst bed. Typically, the catalyst will be initially contacted with a gas containing 0.5% v/v hydrogen or less, the hydrogen content being increased until a hydrogen-rich gas or substantially pure hydrogen is contacting the catalyst.

Thus, a preferred operating regime is to contact the catalyst with an inert gas having a hydrogen content less than 0.5% v/v, most preferably substantially zero, and increasing the hydrogen content of the gas during the activation procedure to a level of from 70% v/v to 100% v/v. It is a particular and most surprising advantage of the process of present invention that complete activation of the catalyst can be achieved using a gas mixture having a final hydrogen content somewhat less than 100% v/v, for example about 75% v/v. In this way, the need for specialized equipment capable of compressing pure hydrogen gas is obviated.

Accordingly in a preferred embodiment of the invention the hydrogen content of the gas mixture is increased from 0.5% v/v or less to 75% v/v during the activation.

The flowrate of the gas mixture is increased during the process of the present invention from an initial rate at the start of activation process. It is a further advantage of the process of the present invention that lower gas flowrates are needed to effect complete activation of the catalyst than with activation processes of the prior art. Thus, the hourly space velocity of the gas (GHSV) may increase within the range from 100 to 10000 Nl/l/h during the process, preferably from 200 to 6000 Nl/l/h. More preferably, the GHSV increases within the range of from 200 to 1500 Nl/l/h. A particularly preferred operating regime for the increasing gas flowrate is to increase the GHSV continuously or stepwise from an initial value of 300 Nl/l/h to a final value of up to 1200 Nl/l/h. An especially preferred operating regime is an increase within the range of from 350 Nl/l/h to up to 1000 Nl/l/h over the duration of the activation process.

The rate at which the flowrate of the gas mixture is increased will be determined by the partial pressure of water in the gas leaving the catalyst bed, which in turn is related to the operating pressure of the process and the rate of increase of the hydrogen partial pressure in the gas contacting the catalyst. In general, higher gas hourly space velocities will be required at a given set of operating conditions for activation of catalysts having a high sensitivity to the presence of water. For the activation of more water-tolerant catalysts, lower gas space velocities and/or higher hydrogen concentrations can be employed.

The activation process of the present invention is carried out at an elevated temperature, preferably below 500 °C. More preferably, the process is operated at a temperature of from 100 °C to 350 °C, especially in the range of from 200 to 300 °C. A preferred temperature for operation of the process is about 250 °C. Conveniently, the temperature is maintained at a constant level throughout the activation procedure. Alternatively, the temperature may be increased stepwise or continuously during the activation, for example from an initial value of 100 °C to a final value of up to 350 °C. More preferably, the temperature is increased within the range of from 200 to 300 °C.

The activation process of the present invention is operated at elevated pressure, typically from 1 to 30 bar, preferably from 1 to 10 bar. More preferably, the catalyst is contacted with the mixture of hydrogen and one or more inert gases at a pressure in the range of from 2 to 8 bar. It will be appreciated that the higher the operating pressure, the higher the partial pressure of any water present in the gas leaving the catalyst bed. An operating pressure of from about 3 to 6 bar is particularly preferred. It is preferred that the pressure of the gas mixture contacting the catalyst is maintained substantially constant throughout the activation process.

The length of time that the catalyst is subjected to the activation process will again depend upon the precise operating conditions and the degree of reduction required, as indicated by the water content of the gas leaving the catalyst bed; a low water content indicating that a high degree of reduction has been achieved. Typically, the process is operated for a period of from 1 to 50 hours, more preferably from 5 to 25 hours.

The process of the present invention may be applied to activate a fresh catalyst, prior to its use in a Fischer-Tropsch synthesis. Alternatively, the process may be applied in the regeneration (reactivation) of an exhausted or partially exhausted catalyst. In this respect, the term "activation" as used herein is to be taken as a reference to the activation of a fresh catalyst prior to its use and to the regeneration (reactivation) of an exhausted or partially exhausted catalyst.

Further, the process of the present invention may be advantageously applied in the reduction/oxidation/reduction, or the so-called "ROR-activation" procedure described in US 4,670,414. The ROR-activation procedure may be advantageously applied in the activation of a fresh catalyst prior to its use, or in the regeneration (reactivation) of an exhausted or partially exhausted catalyst.

Thus, according to a further aspect of the present invention, there is provided a process for the activation of a Fischer-Tropsch catalyst comprising the steps of a) contacting the catalyst with a mixture of hydrogen and one or more inert gases; b) contacting the catalyst with a gas having oxidizing activity; and c) contacting the catalyst with a mixture of hydrogen and one or more inert gases; characterized in that a process as hereinbefore described is employed in at least one of steps a) and c).

Either one or both of the reduction stages in the ROR-activation procedure may comprise the process of the present invention. If the process is employed during only one of the reduction stages of the ROR-activation procedure, it is most preferably used during the second reduction stage.

In the first stage in the ROR-activation procedure, the catalyst particles are contacted with a mixture of hydrogen and one or more inert gases. The process as hereinbefore described may be employed as the first stage in the ROR-activation procedure.

Alternatively, a known reduction procedure may be used, such as described in US 4,670,414. The hydrogen-containing gas used in such a procedure may be substantially pure hydrogen or may comprise hydrogen diluted by one or more inert gases, such as nitrogen. The hydrogen-containing gas may be supplied at a pressure of from 1 to 30 bar, for example about 25 bar. The catalyst is preferably contacted with the gas at a temperature below 500 °C, preferably at a temperature of from 100 to 400 °C, typically from 150 to 350 °C and at a GHSV of from 100 to 10000 Nl/l/h, more preferably from 200 to 6000 Nl/l/h, for a period of from 1 to 50 hours.

The second stage of the ROR-activation procedure is an oxidation stage, in which the catalyst is contacted with a gas having an oxidizing action. The gas used in this stage is conveniently oxygen or an oxygen-containing gas, for example air. The reactions occurring during the oxidation stage are exothermic. In order to avoid an excessive rise in temperature which could damage the catalyst, it is preferred to contact the catalyst with air, further diluted with nitrogen. Typically the gas contains from 1 to 5% v/v, preferably about 3% v/v oxygen. The temperature at which the oxidation is effected is in the range of from 100 to 400 °C, preferably from 150 to 350 °C. The catalyst is contacted with the oxygen-containing gas at a pressure of from 1 to 25 bar, typically about 10 bar, at a GHSV of from 100 to 5000 Nl/l/h, typically from 500 to 1000 Nl/l/h, for a period of from 1 to 30 hours.

The third stage of the ROR-activation procedure is a final reduction of the oxidized catalyst produced in the second stage described above. For this third stage, if the process of the present invention, as hereinbefore described, has been employed in the first stage of the ROR-activation, the known reduction process described above in relation to the first stage may be employed. Most preferably, the process of the present invention is employed in the third stage of the ROR-activation procedure.

In a further aspect, the present invention provides a Fischer-Tropsch catalyst whenever activated by a process as hereinbefore described.

The process of the present invention may be applied as a one-pass process, that is, a process in which the gas mixture fed to the catalyst bed contacts the catalyst only once. In a preferred embodiment, the gas leaving the catalyst bed is dried to reduce the water content of the gas, recompressed to the process operating pressure and recycled to the inlet for the catalyst bed. In a particularly preferred embodiment, the dried, gas being recycled, together with the fresh, mixture of hydrogen and one or more inert gases, is heated by recovering heat from the gas leaving the catalyst bed.

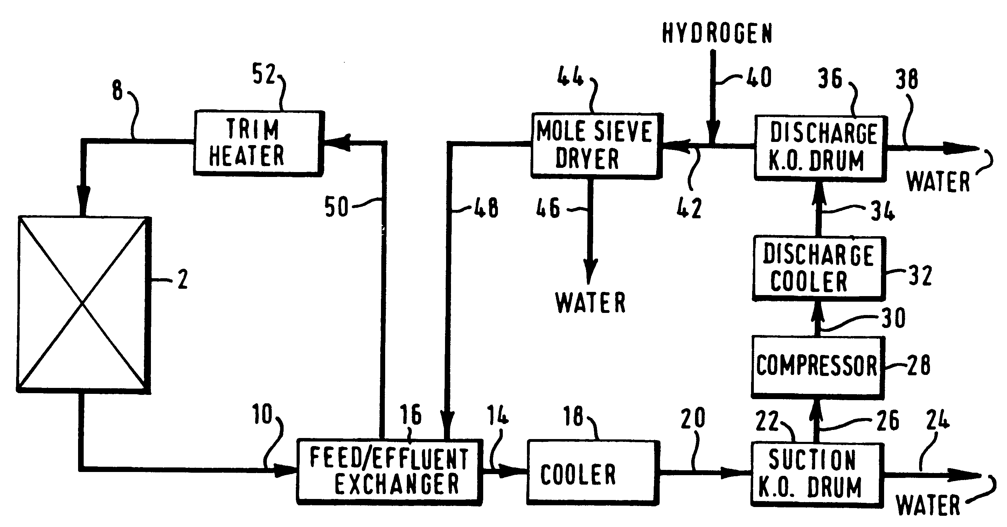

The process of the present invention will be further described with reference to the accompanying figure which is a schematical diagram of a preferred arrangement of apparatus for conducting the process of the present invention.

Referring to the Figure, a reactor vessel 2 has an inlet line 8 and an outlet line 10. Advantageously, fresh catalyst is loaded into a catalyst bed within the reactor 2 prior to being activated by the process of the present invention. The catalyst may also be regenerated (reactivated) whilst remaining in the reactor vessel 2. The catalyst may be regenerated (reactivated) several times whilst remaining in the reactor vessel 2, throughout its useful life.

During the process of the present invention, a mixture of hydrogen and one or more inert gases enters the reactor vessel 2 from the inlet line 8 and contacts the catalyst bed. Effluent gas exiting the catalyst bed leaves the reactor vessel 2 and enters the outlet line 10. The effluent gas in the outlet line 10 is depleted in hydrogen and is rich in water-vapour, relative to the feed gas in the inlet line 8. From the outlet line 10, the effluent gas flows to a feed/effluent heat exchanger 16 to undergo a first stage of cooling and then, via line 14, to a further cooler 18 to undergo a second stage of cooling in preparation for compression. The effluent gas leaves the cooler 18 and, through line 20, enters a suction knock-out drum 22. In the suction knock-out drum 22, water, now present as droplets entrained in the effluent gas, is removed from the effluent gas stream and leaves the suction knock-out drum 22 through line 24. From the suction knock-out drum 22, the effluent gas is fed, via line 26, to the inlet of a compressor 28. The compressed effluent gas leaves the compressor 28 via line 30, is cooled in a discharge cooler 32 and fed, via line 34, to a discharge knock-out drum 36. The action of compression and cooling on the effluent gas stream causes further droplets of water to form, which collect and are removed from the discharge knock-out drum 36 through line 38. From the discharge knock-out drum 36, the effluent gas is mixed with fresh hydrogen-containing gas from line 40 and the combined gas stream fed, through line 42, to a molecular sieve dryer 44.

The molecular sieve dryer 44 may typically contain an aluminosilicate adsorbant for removing water from the combined gas stream. Water is shown, schematically, leaving the molecular sieve dryer 44 through line 46 and the dried gas stream is shown leaving through line 48.

The dried gas stream is heated by heat exchange with the effluent gas leaving the reactor 2 in the feed/effluent exchanger 16. From the feed/effluent exchanger 16, the gas stream is fed, via line 50, to a trim heater 52 to bring the gas up to the process operating temperature. The hot gas enters the reactor 2 from the trim heater 52 through line 8.

The apparatus represented in the Figure may be used to service a plurality of reactor vessels (not shown), each of which is isolated by valves and which may, in turn, be connected to the process apparatus shown in the Figure for activation or regeneration (reactivation) of the catalyst therein.

A bypass line (not shown) may be provided between the two lines 8, 10 to allow gas to bypass the reactor vessel 2 and be removed from the process apparatus as a purge.

The process of the present invention may be applied to any Fischer-Tropsch catalyst. Fischer-Tropsch catalysts frequently comprise, as the catalytically active component, a metal from Group VIII of the Periodic Table of Elements. Particular catalytically active metals include iron, cobalt and nickel. The process of the present invention is particularly advantageous when applied to Fischer-Tropsch catalysts comprising cobalt as the catalytically active metal.

The catalytically active metal is preferably supported on a porous carrier. The porous carrier may be selected from any suitable refractory metal oxide or silicates or a combination thereof. Particular examples of preferred carriers include silica, alumina, titania or mixtures thereof. Most preferably, a porous silica carrier is used. The active metal may be applied to the carrier by any of the techniques well known in the art, for example kneading, impregnation or precipitation. Impregnation is a particularly preferred technique, which may be carried out by contacting the carrier with a compound of the active metal in the presence of a liquid, most conveniently in the form of a solution of the metal compound. The compound of the active metal may be inorganic or organic. Inorganic compounds of the active metal are preferred, in particular nitrates. The liquid used may also be either organic or inorganic, with water being a particularly preferred and convenient liquid.

The amount of catalytically active metal on the carrier is preferably from 3 to 100 pbw per 100 pbw of carrier material, more preferably from 10 to 80 pbw, especially from 20 to 60 pbw.

If desired, the catalyst may also comprise one or more metals or metal oxides as promoters. Suitable metal oxide promoters may be selected from groups IIa, IIIb, IVb, Vb and VIb of the Periodic Table, or the actinides and lanthanides. In particular, oxides of magnesium, calcium, strontium, barium, scandium, yttrium, lanthanum, cerium, titanium, zirconium, hafnium, thesium, uranium, vanadium and chromium are most suitable. A particularly preferred metal oxide promoter is zirconium oxide. Suitable metal promoters may be selected from groups VIIb or VIII of the Periodic Table. Rhenium and group VIII noble metals are particularly suitable, with ruthenium, platinum and palladium being especially preferred. The promoter may be applied to the porous carrier either before or after application of the catalytically active metal. The amount of promoter present is preferably from 0.1 to 150 pbw per 100 pbw of carrier. A particularly preferred catalyst is a cobalt/zirconium/silica catalyst.

Examples of suitable catalysts to which the process of the present invention may be applied are disclosed in European Patent Applications publication Nos. EP 0 104 672, EP 0 110 449, EP 0 127 220, EP 0 167 215, EP 0 180 269 and EP 0 221 598. Examples of very suitable processes for preparing such catalysts are disclosed in UK patent applications Nos. GB 8918845.2 and GB 8925979.0, forming priority for published European patent application publication Nos. EP 0 421 502 and EP 0 428 223 respectively.

The Fischer-Tropsch catalyst, once activated by the process of the present invention is suitable for use in a process for the synthesis of hydrocarbons from a mixture of carbon monoxide and hydrogen, which mixture is commonly referred to as synthesis gas. The conversion of the mixture of hydrogen and carbon monoxide may be carried out at a temperature of from 125 to 350 °C, preferably from 175 to 250 °C, and at a pressure of from 5 to 100 bar, more preferably from 10 to 50 bar. In the process, the catalyst may be contacted with a synthesis gas having a hydrogen to carbon monoxide molar ratio less than 2.5, preferably less than 1.75. More preferably, the hydrogen to carbon monoxide molar ratio of the synthesis gas is in the range of from 0.4 to 1.5, especially from 0.9 to 1.3.

The process of the present invention is further illustrated by the following examples, in which Examples 2 to 5 are activation procedures according to the present invention and Examples 6 to 10 are activation procedures known from the prior art and are for comparative purposes only.

### Example 1 - Catalyst Preparation

Silica spheres (Grade HT-89; 1.5 mm nominal diameter) were loaded into a reactor vessel and dried by contact with nitrogen at a pressure of 4.5 bar whilst heating at a rate of 50 °C/hr to a final temperature of 200 °C. The spheres remained under these final conditions for 2 hours and were subsequently cooled to a temperature below 50 °C. A zirconium-containing solution (Zr(OC₃H₇)₄, 41.7% by weight; n-propanol, 13.9% by weight; acetyl-acetone, 23.3% by weight; toluene, 21.1% by weight) was pumped into the reactor and circulated over the silica spheres for a period of 1.5 hours to allow the bed to reach equilibrium. The reactor was then drained of the solution and the silica spheres flushed with nitrogen at room temperature (20 °C). The solvent remaining in and around the silica spheres was evaporated by heating the spheres to a final temperature of 200 °C in nitrogen at a rate of 50 °C/hr. The spheres were held under these final conditions for 14 hours. After this time, the temperature was again increased, at a rate of 50 °C/hr, to a final temperature of 500 °C and the spheres maintained at this temperature, in an atmosphere of nitrogen, for a period of 1 hour. A mixture of oxygen in nitrogen (0.5 %v O₂) was admitted and the silica spheres held under these conditions for 7 hours. Thereafter, the temperature was increased to 600 °C and the oxygen concentration gradually increased to 2.5 %v for a period of 5 hours. After this time, the spheres were allowed to cool to room temperature.

An aqueous solution of cobalt nitrate (Co(NO₃)₂.6H₂O) having a cobalt concentration of 17.7% by weight was prepared and heated to a temperature of from 60 to 70 °C. The silica spheres prepared above were heated to a temperature of from 90 to 100 °C and immersed in the solution for a period of 30 minutes. The silica spheres were then dried in air at 60 to 70 °C for 7 hours and thereafter calcined by heating at a rate of 35 °C/hr to a temperature of 500 °C, finally being held at this temperature for 1 hour prior to cooling to room temperature.

The resulting catalyst comprised 7.27% by weight zirconium and 17.6% by weight cobalt.

### Examples 2 to 5 - Catalyst Activation and Testing

Four samples of the catalyst prepared in Example 1 were each loaded into a fixed bed reactor and subjected to an activation procedure according to the present invention. The conditions and duration of the activation for each sample are given in Table I. In each activation procedure the conditions were varied so as to maintain a water partial pressure in the off-gas leaving the reactor of below about 50 mbar. The ranges given in Table I for temperature, gas hourly space velocity (GHSV) and hydrogen concentration indicate the starting point and end point for that parameter, the parameter being increased continuously throughout the duration of the activation. The hydrogen gas fed to the reactor in each case was diluted with nitrogen to the indicated hydrogen concentration.

Once activated, each of the catalyst samples was subjected to a hydrocarbon synthesis test, in which the catalyst was contacted with a mixture of carbon monoxide and hydrogen, having a hydrogen/carbon monoxide ratio of about 1.1, at a GHSV of 800 Nl/l/h at a pressure of 26 bars. The operating temperature of the reactor, space time yield (STY) and selectivity to C₅ hydrocarbons and larger (C₅+ selectivity) achieved in each of the tests are given in Table II.

### Comparative Examples 6 to 10

For comparative purposes, five further samples of the catalyst prepared in Example 1 were activated according to procedures known from the prior art. The conditions during activation of the five comparative examples were as described above for Examples 2 to 5, with the exception of those parameters as indicated in Table I.

The five activated catalyst samples were then each subjected to a hydrocarbon synthesis test under the same conditions as described above with reference to Examples 2 to 5. The results of these tests are set out in Table II.

**Table I**

| Example No. | 2 | 3 | 4 | 5 | 6 | 7 | 8 | 9 | 10 |
|---|---|---|---|---|---|---|---|---|---|
| Pressure (bar) | 5 | 5 | 5 | 5 | 3 | 5 | 5 | 3 | 3 |
| Duration (hours) | 50 | 48 | 50 | 50 | 5 | 55 | 75 | 20 | 70 |
| GHSV (Nl/l/h) | 400→600 | 400→600 | 400→600 | 350→500 | 6000 | 400 | 250 | 600 | 600 |
| Hydrogen Concentration (%v) | 1→75 | 1→75 | 1→75 | 1→75 | 1→50 | 1→50 | 1→50 | 100 | 1→100 |
| Temperature (°C) | 240→260 | 240→260 | 260 | 240→260 | 260 | 240 | 240 | 180→260 | 200→260 |

**Table II**

| Example No. | 2 | | | 3 | 4 | | 5 | 6 | 7 | 8 | | 9 | | 10 |
|---|---|---|---|---|---|---|---|---|---|---|---|---|---|---|
| Temperature (°C) | 216 | 216 | 219 | 216 | 220 | 220 | 219 | 213 | 220 | 220 | 224 | 210 | 227 | 210 |
| Duration (hours) | 100 | 300 | 800 | 300 | 400 | 750 | 400 | 300 | 100 | 150 | 400 | 100 | 300 | 100 |
| STY (gC₁+/l/h) | 97 | 94 | 99 | 94 | 102 | 100 | 110 | 96 | 95 | 96 | 98 | 88 | 103 | 100 |
| C₅+ selectivity (%w) | 91 | 90 | 88.5 | 90 | 90 | 90 | 88 | 90 | 88.5 | 90 | 88.5 | 89 | 87 | 88 |

From the data set out in Table II, it can be seen that, in general, a catalyst activated using a procedure according to the present invention (Examples 2 to 5) exhibits an excellent overall activity (as indicated by a low synthesis temperature) giving high space time yields with a very high selectivity to C₅+ hydrocarbons.

From a comparison of the performance of the catalyst activated by procedures according the present invention with the same catalyst activated following procedures known from the prior art (Examples 6 to 10), the following will be realized:

As shown in Example 6, a very high gas space velocity during activation yielded a catalyst having an excellent activity (low synthesis temperature), a high yield and a very high C₅+ selectivity. This high level of catalyst performance was achieved at gas space velocities of no more than 10% the value used in Example 6 by applying the process of the present invention. By comparison, activation using a constant gas space velocity of 10% or less than that of Example 6 (Examples 7 to 10) yielded a catalyst having a significantly lower yield, activity and selectivity.

Further, in general the activation procedure of the present invention required considerably less time to achieve a high degree of catalyst activation than equivalent processes of the prior art.

Finally, as shown in Tables I and II, the activation procedure of the present invention did not require the use of pure hydrogen to achieve a high degree of catalyst activation.

## Claims

1. A process for the activation of a Fischer-Tropsch catalyst comprising reducing the catalyst by contact with a mixture consisting of hydrogen with one or more inert gases, the hydrogen concentration expressed as % volume hydrogen / volume gas mixtur and the space velocity of the gas expressed as Nl/l/h contacting the catalyst increasing step-wise or continuously during the activation, wherein the hydrogen content of the gas mixture is increased from 0.5% v/v or less to 100% v/v.

2. A process according to claim 1, wherein the water partial pressure in the gas leaving the catalyst is maintained below 200 mbar, preferably below 100 mbar.

3. A process according to any preceding claim, wherein the hydrogen content of the gas mixture is increased from 0.5% v/v or less to 75% v/v during the activation.

4. A process according to any preceding claim, wherein the space velocity is increased within the range of from 100 to 10000 Nl/l/h, preferably within the range of from 200 to 6000 Nl/l/h, more preferably within the range of from 300 Nl/l/h up to 1200 Nl/l/h.

5. A process according to any preceding claim, wherein the pressure of the gas mixture is substantially constant during the activation.

6. A process according to any preceding claim, wherein the pressure of the gas mixture is in the range of from 1 to 10 bar.

7. A process according to any preceding claim, wherein the activation is effected at a temperature in the range of from 100 to 350°C.

8. A process according to any preceding claim, wherein the gas leaving the catalyst is dried, combined with a fresh mixture of hydrogen with one or more inert gases and recycled to further contact the catalyst.

9. A process for the activation of a Fischer-Tropsch catalyst comprising the steps of
a) contacting the catalyst with a mixture of hydrogen with one or more inert gases:
b) contacting the catalyst with a gas having oxidizing activity; and
c) contacting the catalyst with a mixture of hydrogen with one or more inert gases,
wherein a process as defined in any one of claims 1 to 8 is employed in at least one of steps a) and c), preferably in at least step c).

10. A process according to any preceding claim, characterized in that the catalyst comprises silica, alumina, titania or mixtures thereof as carrier.

11. A process according to any preceding claim, characterized in that the catalyst comprises cobalt as a catalytically active metal.

12. A process according to any preceding claim, characterized in that the catalyst comprises as metal oxide promoter zirconium oxide

13. A Fischer-Tropsch catalyst activated by a process according to any preceding claim

14. A process for the preparation of hydrocarbons comprising contacting a mixture of carbon monoxide and hydrogen with a catalyst according to claim 13.

## Patentansprüche

1. Verfahren zur Aktivierung eines Fischer-Tropsch-Katalysators, bei dem man den Katalysator durch Inberührungbringen mit einem aus Wasserstoff und einem oder mehreren Inertgasen bestehenden Gemisch reduziert, wobei man während der Aktivierung die Wasserstoffkonzentration, ausgedrückt als Vol.-% Wasser-Stoff/Volumen Gasgemisch, und die Raumgeschwindigkeit des mit dem Katalysator in Berührung kommenden Gases, ausgedrückt als Nl/l/h, schrittweise oder kontinuierlich erhöht, wobei der Wasserstoffgehalt des Gasgemisches von 0,5 Vol.-% oder weniger auf 100 Vol.-% erhöht wird.

2. Verfahren nach Anspruch 1, bei dem man den Wasserpartialdruck des den Katalysator verlassenden Gases unter 200 mbar, vorzugsweise unter 100 mbar, hält.

3. Verfahren nach einem der vorhergehenden Ansprüche, bei dem man während der Aktivierung den Wasserstoffgehalt des Gasgemisches von 0,5 Vol.-% oder weniger auf 75 Vol.-% erhöht.

4. Verfahren nach einem der vorhergehenden Ansprüche, bei dem man die Katalysatorbelastung im Bereich von 100 bis 10000 Nl/l/h, vorzugsweise im Bereich von 200 bis 6000 Nl/l/h, besonders bevorzugt im Bereich von 300 Nl/l/h bis zu 1200 Nl/l/h, erhöht.

5. Verfahren nach einem der vorhergehenden Ansprüche, bei dem man während der Aktivierung den Druck des Gasgemisches weitgehend konstant hält.

6. Verfahren nach einem der vorhergehenden Ansprüche, bei dem man den Druck des Gasgemisches im Bereich von 1 bis 10 bar hält.

7. Verfahren nach einem der vorhergehenden Ansprüche, bei dem man bei einer Temperatur von 100 bis 350°C aktiviert.

8. Verfahren nach einem der vorhergehenden Ansprüche, bei dem man das den Katalysator verlassende Gas trocknet, mit einem frischen Gemisch aus Wasserstoff und einem oder mehreren Inertgasen vermischt und zum weiteren Kontakt mit dem Katalysator zurückführt.

9. Verfahren zur Aktivierung eines Fischer-Tropsch-Katalysators, das folgende Schritte umfaßt:
a) Inberührungbringen des Katalysators mit einem Gemisch aus Wasserstoff und einem oder mehreren Inertgasen;
b) Inberührungbringen des Katalysators mit einem oxidationsaktiven Gas, und
c) Inberührungbringen des Katalysators mit einem Gemisch aus Wasserstoff und einem oder mehreren Inertgasen,
bei dem man in mindestens einem der Schritte a) und c), vorzugsweise mindestens in Schritt c), ein Verfahren nach einem der Ansprüche 1-8 anwendet.

10. Verfahren nach einem der vorhergehenden Ansprüche, dadurch gekennzeichnet, das der Katalysator Siliziumoxid, Aluminiumoxid und Titanoxid oder deren Gemische als Träger enthält.

11. Verfahren nach einem der vorhergehenden Ansprüche, dadurch gekennzeichnet, daß der Katalysator Kobalt als katalytisch aktives Metall enthält.

12. Verfahren nach einem der vorhergehenden Ansprüche, dadurch gekennzeichnet, daß der Katalysator Zirkoniumoxid als Metalloxidpromotor enthält.

13. Fischer-Tropsch-Katalysator, aktiviert nach einem Verfahren nach einem der vorhergehenden Ansprüche.

14. Verfahren zur Herstellung von Kohlenwasserstoffen, bei dem man ein Gemisch aus Kohlenmonoxid und Wasserstoff mit einem Katalysator nach Anspruch 13 in Berührung bringt.

## Revendications

1. Procédé pour l'activation d'un Fischer-Tropsch catalyseur comprenant la réduction du catalyseur par mise en contact avec un mélange formé d'hydrogène et d'un ou plusieurs gaz inertes, la concentration d'hydrogène exprimée en % en volume d'hydrogène/volume de mélange gazeux et la vitesse spatiale du gaz exprimée en Nl/l/h en contact avec le catalyseur augmentant par incrément ou de manière continue pendant l'activation, dans lequel la teneur en hydrogène du mélange gazeux est augmentée de 0,5% en volume par volume ou moins à 100% en volume par volume.

2. Procédé selon la revendication 1, caractérisé en ce que la pression partielle d'eau dans le gaz quittant le catalyseur est maintenue au-dessous de 200 mbar, de préférence au-dessous de 100 mbar.

3. Procédé selon l'une quelconque des revendications précédentes, caractérisé en ce que la teneur en hydrogène du mélange gazeux est augmentée de 0,5 % en volume par volume ou moins à 75 % en volume par volume pendant l'activation.

4. Procédé selon l'une quelconque des revendications précédentes, caractérisé en ce que la vitesse spatiale est augmentée dons le domaine de 100 à 10 000 Nl/l/h, de préférence dans le domaine de 200 à 6 000 Nl/l/h, plus préférablement dans le domaine de 300 à 1 200 Nl/l/h.

5. Procédé selon l'une quelconque des revendications précédentes, caractérisé en ce que la pression du mélange gazeux est substantiellement constante pendant l'activation.

6. Procédé selon l'une quelconque des revendications précédentes, caractérisé en ce que la pression du mélange gazeux est dons le domaine de 1 à 10 bar.

7. Procédé selon l'une quelconque des revendications précédentes, caractérisé en ce que l'activation est effectuée à une température dans le domaine de 100 à 350°C.

8. Procédé selon l'une quelconque des revendications précédentes, caractérisé en ce que le gaz quittant le catalyseur est séché, combiné avec un mélange frais d'hydrogène avec un ou plusieurs gaz inertes, et recyclé pour une mise en contact supplémentaire avec le catalyseur.

9. Procédé pour l'activation d'un Fischer-Tropsch catalyseur comprenant les étapes de
a) mise en contact du catalyseur avec un mélange d'hydrogène avec un ou plusieurs gaz inertes;
b) mise en contact du catalyseur avec un gaz ayant une activité oxydante;
c) mise en contact du catalyseur avec un mélange d'hydrogène avec un ou plusieurs gaz inertes, caractérisé en ce que l'on emploie un procédé tel qu'il est défini dans l'une quelconque des revendications 1 à 8 dans au moins une des étapes a) et c), de préférence dans au moins l'étape c).

10. Procédé selon l'une quelconque des revendications précédentes, caractérisé en ce que le catalyseur comprend de la silice, de l'alumine, de l'oxyde de titane ou des mélanges de ces derniers en tant que support.

11. Procédé selon l'une quelconque des revendications précédentes, caractérisé en ce que le catalyseur comprend du cobalt en tant que métal catalytiquement actif.

12. Procédé selon l'une quelconque des revendications précédentes, caractérisé en ce que le catalyseur comprend, en tant que promoteur d'oxy les métalliques, de l'oxyde de zirconium.

13. Fischer-Tropsch catalyseur activé par un procédé selon l'une quelconque des revendications précédentes.

14. Procédé pour la préparation d'hydrocarbures comprenant la mise en contact d'un mélange de monoxyde de carbone et d'hydrogène avec un catalyseur selon la revendication 13.
